# EUROPEAN PATENT APPLICATION

(11) **EP 2 859 909 A1**
(43) Date of publication of application: **15.04.2015**
(21) Application number: 13803520.9
(22) Date of filing: 24.01.2013
(51) Int. Cl.: A61M 37/00, A61M 5/158

(54) **SELF-INJECTION MECHANISM FOR USE ON SKIN**

(30) Priority: 12.06.2012 KR 20120062714; 22.11.2012 KR 20120132763
(71) Applicant: JM Biotech Co., Ltd, Daegu 704-23 (KR)
(72) Inventor: CHANG, Ji Young, Daegu 704-872 (KR)
(74) Representative: Peters, Sebastian Martinus
(86) International application number: PCT/KR2013/000561
(87) International publication number: WO 2013/187572

(57) **Abstract**

The present invention relates to a self-injection mechanism that causes a solution (2) having predetermined efficacy to permeate the skin (1) for skin improvement and beauty treatment. The present invention includes: an accommodation unit (10) having a space (11) therein for accommodating the solution (2), an inlet (12) which sucks only air at one end, and an outlet (15) which is open so that the solution (2) is discharged at the other end; a pumping unit (20) coupled to the outlet (15) of the accommodation unit (10) so as to be in communication with the same, having an airless pump (21) which discharges the solution (2) by an up and down movement at the center; and an injection unit (30) coupled with the airless pump (21) of the pumping unit (20) so as to be in communication with the same, causing the solution (2) which is discharged by the up and down movement to permeate the skin (1). The present invention can be formed into a stamp-type simple structure in order to reduce manufacturing costs, and thus can be used easily and conveniently at home at inexpensive cost. Also, the solution can be injected directly and uniformly by a needle penetrating the outer skin layer and the thick skin layer of the skin so as to minimize loss of the solution while maximizing the efficacy thereof.

## Description

### Technical Field

The present invention relates to a self-injection mechanism for a skin improvement or a beauty care by penetrating, into a skin, a solution which has predetermined efficacy, and in particular to a self-injection mechanism for the use on a skin which is characterized by forming a stamp-type structure, which is light, thin, short and small, thereby lowering a manufacturing cost. Accordingly, every home can easily and conveniently equip the mechanism at a lower cost, and the efficacy may be maximized while minimizing any loss of a solution in such a way to uniformly and directly inject the solution in a state where a needle is penetrated inside an epidermis and a dermis of a skin.

### Background Art

A human skin in general is divided into an epidermis (outer skin), a dermis (inner skin), and a subcutaneous tissue. In case of a healthy and young skin, about 80% of the dermis is filled with collagen and elastin. In case of the dermis, the quantities of the collagen and the elastin gradually decrease (contraction) as time goes by, so the dermis becomes thinner, and the skin becomes dry and wrinkles increase, so the elasticity of the skin is lowered. Therefore, various nutritious substances, for example, a substance (functional cosmetics) such as vitamin C, peptide, etc., which help the creations of the collagen, elastin, etc. are coated on the skin in order to maintain the skin elastic while preventing the loss of the elasticity.

However, the quantities of the nutritious substances which penetrate through the epidermis into the dermis account for about 0.3%, which is very small quantity. Since the way where the above-mentioned various nutritious substances are simply coated on the epidermis of the skin have very weak effects, it urgently needs to develop a predetermined mechanism which may help effectively penetrate the above nutritious substances into the dermis which resides deep inside the skin without any loss.

For instance, according to the Korean Utility Model Registration No. 20-0395359 entitled "micro-needle roller", the micro-needle roller is characterized in that the roller is formed by laminating at least one circular disk, and at least one micro-needle is disposed inside the circular disk or at one surface thereof, and a predetermined length of one end of the thusly disposed micro-needle exposes from the outer surface of the roller.

For another instance, according to the Korean Utility Model Registration No. 20-0423963 entitled "stamp type skin stimulator", the stamp type skin stimulator includes a hexahedron shaped housing the top of which is open, a handle bar formed of a rod downwardly extending from the bottom of the housing, a flat plate-shaped cover which is secured to the upper surface of the housing and includes a plurality of needle holes arranged at regular intervals, and a plurality of needles which fit into the needle holes wherein the ends of the needles expose from the needle holes.

The mechanism mentioned in the above prior has an advantage in the way that a penetration force of the nutritious substance into the dermis may be a little increased since a needle artificially forms the hole passing through the epidermis and the dermis of the skin in such a way that a needle having a predetermined protruding length is disposed at an outer circumferential surface of the roller and on one surface of the stamp, and then the roller and the stamp are contacted with the skin; however in case of the roller, since the needle does not vertically contact with the skin, the epidermis and the dermis may be hurt, thus doubling the user's pain. In particular, in case of the head skin or the skin which has lots of hairs or body hairs, the body hairs may get tangled between the needles.

Here, in case of the stamp, it improves the problem where the user feels pain since the needle vertically contacts with the skin and the body hairs get entangled, but since the nutritious substance is coated on the surface of the epidermis after the artificial holes of the epidermis and the dermis like the roller are formed, a very small quantity of the substance reaches (absorbed) the dermis. Even though the needle artificially forms the holes passing through the epidermis and the dermis, since the skin of the human basically has a barrier protection function for protecting from the outside, the holes are suddenly closed. For example, in case where the skin is burned, the patient does not die of the hurt skin, but the patient dies of infections since various bacteria penetrate from the outside into the skin because the hurt skin loses the barrier function. Therefore, there may be a limit when effectively penetrating the nutritious substance into the dermis without any loss by forming only the holes through the epidermis and the dermis of the skin.

In addition to that, according to the Korean Patent Publication No. 10-2011-0108445 entitled "automatic skin micro-channel stamp which helps acne, various scars and wrinkle therapy", a stamp head 106a is attached to a body 103a, and the height of the head may be adjustable based on a length adjusting groove 103b (the length where the needle exposes is therefore determined), and a thin needle surface 107a quickly forms a fine hole at the skin and separates from there by inserting a spring 105a into a body. Disclosed is the automatic skin micro-channel stamp in which the depth where the needle separates from the stamp head 106a contacting with the skin and is penetrated into the skin may be adjusted within a range of 0.20mm∼2.3mm.

The mechanism mentioned in the prior art is characterized in that the needle is hidden inside the stamp, and the penetration depth of the needle may be adjusted. In the structure, the stamp closely contacts with the skin, and the user presses the operable unit with hands, and the needle protrudes to artificially form the holes passing through the epidermis and the dermis of the skin, and it recovers by means of a spring. The above mentioned mechanism however improves only a part as compared with the earlier mentioned stamp mechanism, and since there is not a fundamental improvement to enhance the penetration force of the nutritious substance, the same problems occur. Namely, even though the needle artificially forms the holes passing through the epidermis and the dermis, since the skin of the human basically has a barrier function for protecting from the outside, the holes are suddenly closed. Therefore, there is a limit when effectively penetrating the nutritious substances into the dermis without any loss only by simply forming the holes passing through the epidermis and the dermis of the skin.

Meanwhile, in recent years, various skin care tools are being used for surgeries at dermatology. Among such tools, there are assistant tools such as a cellas which uses laser and a mesogun which uses needle wherein the above tools are used for forming small holes on a skin. The cellas is a laser tool characterized in that about 2,000∼10,000 holes are uniformly formed using laser beam at the depths of 0.1mm∼1.5mm at the intervals of 0.3mm∼1.0mm, and new flesh is guided to grow from the surrounding living cells where the laser beam did not pass through, to the dead cells where the laser beam passed through, and the mesogun is a gun type medical tool with a small needle, more specifically, a medical tool which is capable of improving absorption rate by injecting medicine and cosmetic after forming holes using the needle up to the dermis of the skin. Such medical tools provide effects for helping the dermis therapy and improving the reaches of the functional cosmetic and active up to the dermis; however the above medical tools are so bulky, and prices are high, and the products are expensive. Above all, it is impossible to use without the medical doctor's accurate diagnosis and surgery after the patients visit the dermatology.

### Detailed Description of the Invention

### Disclosure of Invention

Accordingly, the present invention is to fundamentally solve the problems of the conventional art. It is an object of the present invention to provide a self-injection mechanism for the use on a skin characterized in that since the mechanism is formed in a stamp type light, thin, short and small structure, the manufacturing cost may become lower, and every home may easily and conveniently adopt for lower prices, and any loss of the solution may be minimized in such a way to uniformly and directly inject the solution in a state where the needle has penetrated in the epidermis and the dermis of the ski while maximizing the efficiency.

### Means for Solving the Problem

To achieve the above objects, according to one aspect of the present invention, there is provided a self-injection mechanism for use on skin configured to inject a solution having predetermined efficacy into a skin for skin improvements or beauty treatment, which includes an accommodation unit which includes a space configured to accommodate the solution inside the space, a suction port disposed at one end for sucking only air, and a discharge port disposed at the other end and being open for discharging the solution; a pumping unit which is engaged to communicate with the discharge port of the accommodation unit and includes an airless pump disposed at the center for discharging the solution based on up and down motions; and an injection unit which is engaged to communicate with the airless pump of the pumping unit and is configured to penetrate the solution discharged based on the up and down motions into the skin.

At this time, the accommodation unit includes a cover with a suction hole being interposed together with a packing which floats over the space while sealing the solution of the suction port.

In addition, the injection unit of the present invention includes a housing which includes a fixture and a slanted member combined with the airless pump; a needle unit which includes a needle groove and a needle disposed at the bottom of the housing; and a fixing unit which includes a fixing groove and a fixing protrusion for fixing the housing and the needle unit.

Also, the housing of the present invention further includes a rubber grip or a protrusion formed on an outer circumferential surface for improving a user's grip feeling and preventing slip by a user.

Also, the housing of the present invention further includes at least one fixing protrusion and slide groove on the top of an inner circumferential surface for preventing the rotations from the pumping unit.

To achieve the above objects, according to another aspect of the present invention, there is provided a self-injection mechanism for use on skin configured to inject a solution having predetermined efficacy into a skin for skin improvements or beauty treatment, which includes a handle unit which protrudes for a user to grab and includes an engaging protrusion having at least one shoulder at the bottom; a packing unit which is detachably disposed at the bottom of the handle unit and includes an engaging groove fixedly engaged with the engaging protrusion; and an injection unit which is sealingly engaged with the packing unit and is configured to discharge and penetrate the solution into the skin based on the pressurizing motions of the handle unit.

At this time, the handle unit of the present invention includes a rubber grip or a protrusion on its outer circumferential surface so as to improve grip feeling and prevent slip.

Also, the injection unit includes a housing which is combined with the packing unit and includes a fixture, a slanted member and a space for accommodating the solution; a needle unit which includes a needle groove and a needle at the bottom of the housing; and a fixing unit which includes a fixing groove and a fixing protrusion for fixing the housing and the needle unit.

Also, according to one aspect or another aspect of the present invention, the needle groove of the needle unit includes a fixing groove for fixedly inserting the needles; and a guide groove for guiding, in safe, for the needles to move to the center over the top of the fixing groove.

Also, according to one aspect or another aspect of the present invention, the needle unit includes a plurality of needles which protrude by predetermined distances long enough to penetrate into the skin, and at least one of the plurality of the needles is a non-protruding needle which comes into contact with the surface of the skin.

Also, according to one aspect or another aspect of the present invention, the fixing unit is configured to fix the needle onto the slanted member of the housing and further fills a hardener to minimize the consumption of the solution by blocking the space.

Also, according to one aspect or another aspect of the present invention, the housing further includes a safety cap with an engaging protrusion being interposed so as to block the inputs of bacteria or impurities while protecting the needle unit.

As the present invention may be embodied in several forms without departing from the spirit or essential characteristics thereof, it should also be understood that the above-described examples are not limited by any of the details of the foregoing description, unless otherwise specified, but rather should be construed broadly within its spirit and scope as defined in the appended claims. Therefore all changes and modifications that fall within the meets and bounds of the claims, or equivalences of such meets and bounds are intended to be embraced by the appended claims.

### Advantageous effects

As described in the above configuration and operation, the present invention is characterized in that since the mechanism is formed in a stamp type structure which is light, thin, short and small, the manufacturing cost may become lower, and every home may easily and conveniently adopt for lower prices, and any loss of the solution may be minimized in such a way to uniformly and directly inject the solution in a state where the needle has penetrated in the epidermis and the dermis of the skin while maximizing the efficiency.

### Brief Description of Drawings

Figure 1 is a perspective view illustrating an injection mechanism in whole according to one aspect of the present invention.
Figure 2 is a disassembled view illustrating a configuration after an injection mechanism is disassembled according to one aspect of the present invention.
Figure 3 is a disassembled view illustrating a configuration after the major components of an injection mechanism are disassembled according to one aspect of the present invention.
Figure 4 is a cross sectional view illustrating a configuration after an injection mechanism is cut-away according to one aspect of the present invention.
Figure 5 is a constructional view illustrating a configuration of an exemplary embodiment according to one aspect of the present invention.
Figure 6 is a constructional view illustrating a configuration of a modified example according to one aspect of the present invention.
Figure 7 is a cross sectional view illustrating a configuration after an injection mechanism is in whole cut-away according to one aspect of the present invention.
Figure 8 is a disassembled view illustrating a configuration after the major components of an injection mechanism are disassembled according to another aspect of the present invention.
Figure 9 is a constructional view illustrating a configuration of an exemplary embodiment according to another aspect of the present invention.
Figures 10 to 12 are views illustrating an injection state so as to show a use state of an injection mechanism of the present invention.
Figure 13 is a schematic view schematically illustrating a use state of an injection mechanism according to the present invention.

### Best modes for carrying out the invention

The exemplary embodiments of the present invention will be described in detail with reference to the accompanying drawings.

The present invention relates to a self-injection mechanism for improving or caring skin by penetrating a solution 2, which has predetermined efficacy, into a skin 1. In one aspect of the present invention, the present invention is directed to a self-injection mechanism for the use on a skin, characterized in that the self-injection mechanism comprises an accommodation unit 10, a pumping unit 20 and an injection unit 30, as major components. In the other aspect of the present invention, the self-injection mechanism comprises a handle unit 10, a packing unit 20 and an injection unit 30, as major components. Namely, the same injection unit 30 is used for both the one aspect and the other aspect of the present invention. The accommodation unit 10 and the pumping unit 20 or the handle unit 10 and the pacing unit 20, which cooperate with the injection unit 30, may be selectively used. Hereinafter, the present invention will be described based on the exemplary embodiments.

The term "self-injection mechanism" represents a new concept medicine transfer mechanism which enables to inject the solution 2 in a state of forming a new passage directly passing through the epidermis 1a and the dermis 1b of the skin 1. Namely, the self-injection mechanism is an anti-aging solution mechanism which is characterized in that since the self-injection mechanism is provided in a state where the solution 2 having outstanding effects for the skin improvements and caring verified at the ministry of food and drug safety is previously filled, the user may inject by himself at home like applying the cosmetic, so it is possible to secure the same effects as or better effects than the laser-based peeling surgery while preventing any hurts at the epidermis 1a of the skin 1 thanks to the natural therapy of the injuries.

Meanwhile, the solution 2 mentioned in the present invention uses components such as hyaluronic acid, vitamin C, collagen, elastin, arbutin, kojic acid, peptide, etc. which are all related to the skin improvement and caring. In addition to that, various components may be used, and any components which will be developed may be also used. It is preferred that the solution which needs a consultation or a prescription from a medical doctor or is classified as a medical medicine for a skin disease, etc. should not be used. In any case, it is obvious that a person having ordinary skill in the art may provide various methods and components within a range that has not violated any medical act.

### First Exemplary Embodiment

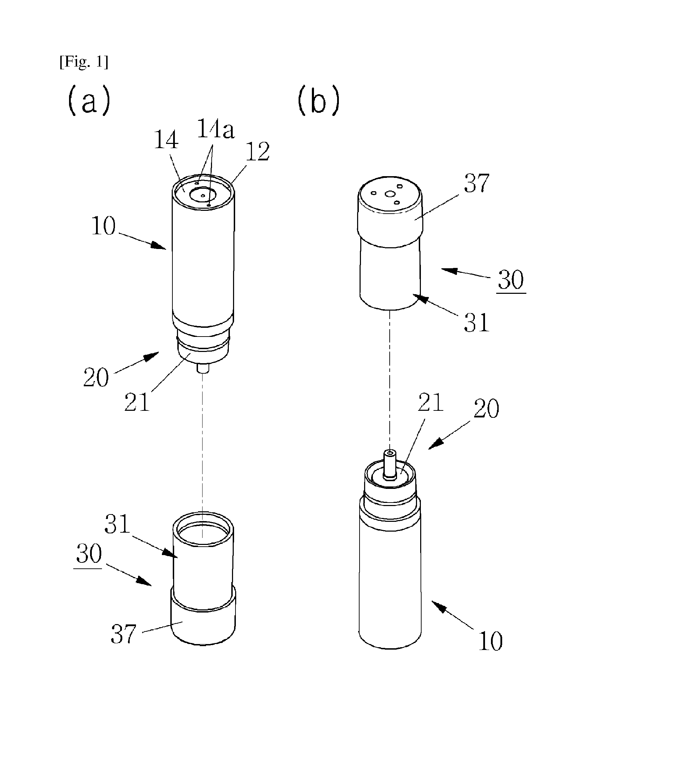

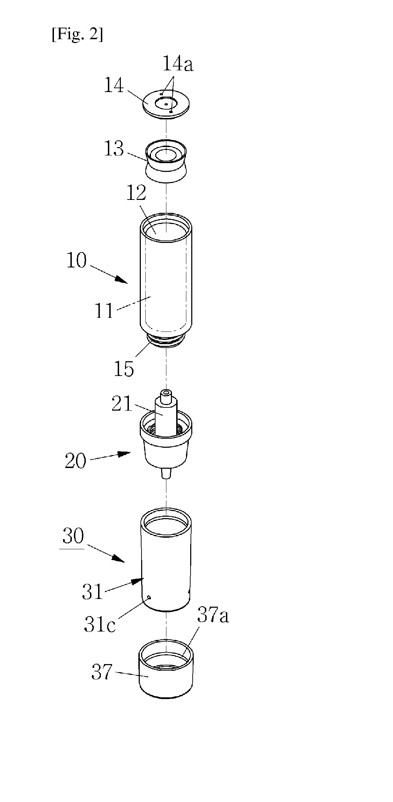

The accommodation unit 10 according to the present invention includes a space 11 for storing the solution 2 inside the space 11, a suction port 12 formed at one end for sucking only air, and a discharge port 15 disposed at the other end and being open for discharging the solution 2. Referring to Figures 1 and 2, the accommodation unit 10 is a container configured to accommodate and discharge the solution 2 having the effects good for the skin improvements and caring and has a space 11 with a predetermined volume for storing the solution 2 in safe inside the space 11.

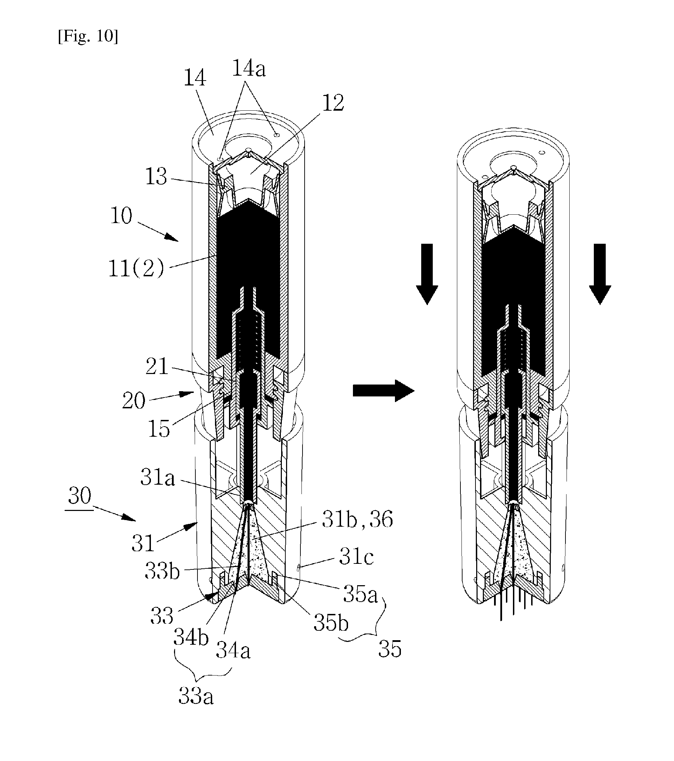

At this time, the accommodation unit 10 according to the present invention includes a cover 14 with a suction hole 14a being interposed together with a packing 13 which floats over the space 11 while sealing the solution 2 of the suction port 12f. Referring to Figure 10, the suction port 12 is configured in order for the solution 2 stored in the space to be sealed from the outside and includes a cover 14 together with the packing 13 for sucking only air so that the solution 2 may be discharged through the discharge port 15 by means of the pumping unit 20, which will be described later.

The packing 13 is made from a smooth rubber material and serves to prevent the solution 2 from being discharged to the outside by completely sealing the suction port 12 of the accommodation unit 1, and the cover 14 is fixedly secured to the suction port 12 of the accommodation unit 10 and includes at least one suction hole 14a at its center for sucking air. Namely, referring to Figure 10, when the solution 2 is discharged though the discharge port 15 by means of the pumping unit 20, the packing 13 floats downward by as much as the volume of the solution 2 discharged based on the difference in pressure, and the air is sucked into the suction hole 14a of the cover 14 by means of the floated packing 13. The solution 2 may be discharged while sealing from the outside the inside of the space 11 with the aid of the packing 13 disposed at the suction port 12 and the cover 14. Here, the discharge port 15 protrudes by a predetermined distance long enough for the pumping unit 20 to be secured and includes a spiral portion on its outer circumferential surface in order for the pumping unit 20 to be detachably attached from the accommodation unit 10. As not illustrated in the drawings, in case where the pumping unit 20 is integrally formed so that the pumping unit 20 may be impossible to detachably attach from the accommodation unit 10, the spiral portion may be omitted. The above-mentioned integral or separate type will be described in detail later along with the pumping unit 20.

The pumping unit 20 according to the present invention is secured to communicate with the discharge port 15 of the accommodation unit 10 and includes at its central portion an airless pump 21 configured to discharge the solution 2 based on up and down movements. Referring to Figures 1 and 2, the pumping unit 20 is secured to the discharge port 15 of the accommodation unit 10 to discharge to the outside the solution 2 accommodated in the space 11 and includes inside of the same an airless pump 21 configured to discharge the solution 2 with a predetermined pressure.

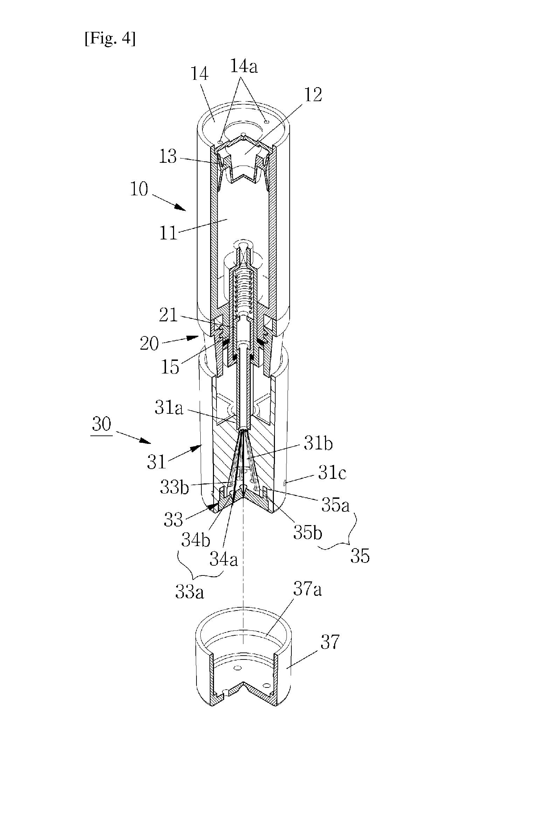

Referring to Figure 4, the pumping unit 20 includes a casing secured to the discharge port of the accommodation unit 10 and includes inside of the same an airless pump 21. The airless pump 21 as a whole includes a cylinder elastically supported together with one direction valve, and a nozzle which floats upwardly and downwardly receiving an elastic force of the spring inside the cylinder. Referring to Figure 10, the solution 2 flows through the valve disposed at one end of the cylinder into the interior of the cylinder, and the inputted solution 2 cannot be discharged again due to the presence of the valve. In this state, when the nozzle floats upward, the volume of the inputted solution 2 decreases and moves into the interior of the nozzle and is completely discharged to the outside. In addition, the nozzle recovers by the spring and allows the solution to flow again into the interior of the cylinder.

Here, a spiral groove is formed on an inner circumferential surface of the casing to engage with the spiral portion of the discharge port, and one end is sealed to engage with one surface of the discharge port 15 in a communicative and sealing way. Of course, the accommodation unit 10 and the pumping unit 20 are integral types like the earlier described discharge port 15, the spiral groove may be omitted, provided that for integrally engaging such components, the casing is bent toward an outer circumferential surface of the discharge port 15.

Meanwhile, when the accommodation unit 10 and the pumping unit 20 are formed in the separate type, the user can separate the accommodation unit 10 and the pumping unit 20 and then can refill the solution, but when such components are formed in the integral type, they should be discarded (recycle) after the solution 2 runs out. Namely, it is preferred that the accommodation unit 10 and the pumping unit 20 are formed in the integral type for safety in terms of the infection of bacteria, but there may be no problems even if they are refilled and used based on the compositions and use range of the solution 2. Above all, even though they are all formed in the integral type or the separate type and are used in a disposable type, since the accommodation unit 10 and the pumping unit 20 may be easily detached (separated) in case of the separation type during the recycling procedure, the process may be greatly shortened. In any case, it is obvious that a person having ordinary skill in the art may selectively adopt and provide them based on the most efficient way in consideration of the process performance based on the manufacturing and recycling and the convenience when in use.

In addition, the injection unit 30 according to the present invention is communicatively combined with the airless pump 21 of the pumping unit 20 and allows the solution 2, which is discharged based on the up and down movements, to penetrate into the skin 1. Referring to Figures 1 and 2, the injection unit 30 is configured to penetrate the solution, which is discharged by the pumping unit 20, into the skin 1 and is communicatively combined with the airless pump 21.

At this time, the injection unit 30 according to the present invention includes a housing 31 formed of a fixture 31a and a slanted member 31b both combined with the airless pump 21, a needle unit 33 formed of a needle groove 33a and a needle 33b at the bottom of the housing 31, and a fixing unit 35 formed of a fixing groove 35a and a fixing protrusion 35b both serving to fix the needle unit 33.

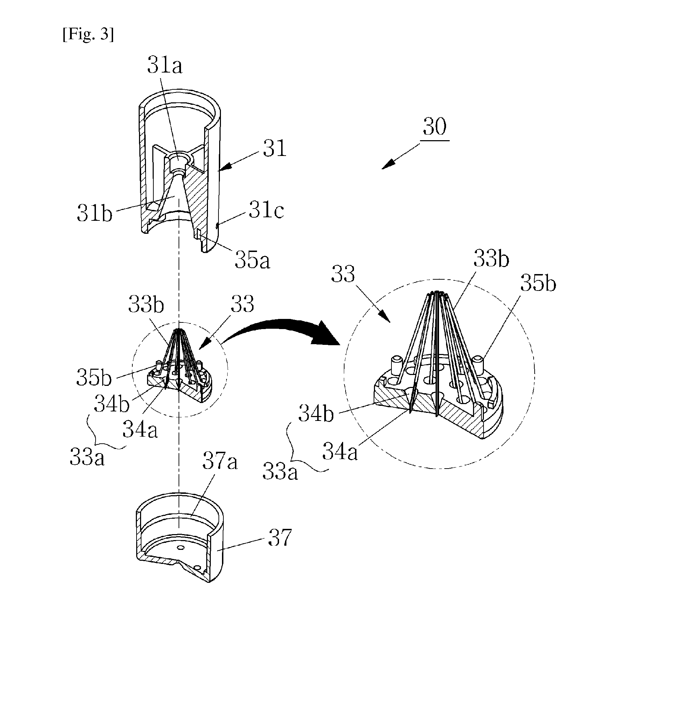

Referring to Figures 3 and 4, the housing 31 is configured to guide and discharge the solution 2 stored in the accommodation unit 10 to the skin 1 and is engaged to communicate with one end of the pumping unit 20 while maintaining a sealed state. Namely, referring to Figure 3, the housing 31 integrally includes a fixture 31a having a two-stepped shoulder for the sake of a tight fit (attachment and detachment) with one end of the airless pump 21, and the fixture 31a guides for the solution 2 to be discharged by moving upwardly and downwardly the airless pump 21 in such a way to transfer a pressurizing force from the user. In addition, the housing 31 integrally includes a slanted member 31b which extends at a predetermined angle to the bottom of the fixture 31a. The slanted member 31b provides a space for accommodating the needle 33b in cooperation with the needle unit 33. Here, the reason why the slanted member 31b is obliquely formed at a predetermined angle is that the needle 33b can guide the solution 2, which is discharged from the airless pump 21, to be distributed with the same quantity. This operation will be described in detail later. Of course, according to the situation given, any modification to form a straight member instead of the slanted member 31b is not eliminated.

At this time, the housing 31 according to the present invention further includes a rubber grip 32a or a protrusion 32b on an outer circumferential surface for preventing slip while improving grip feeling. The housing 31 is configured to discharge the solution 2 stored in the accommodation unit 10 to the skin 1 and includes on its outer circumferential surface a rubber grip 32a or a protrusion 32b for the sake of safe injection in a state where the user is grabbing with hands.

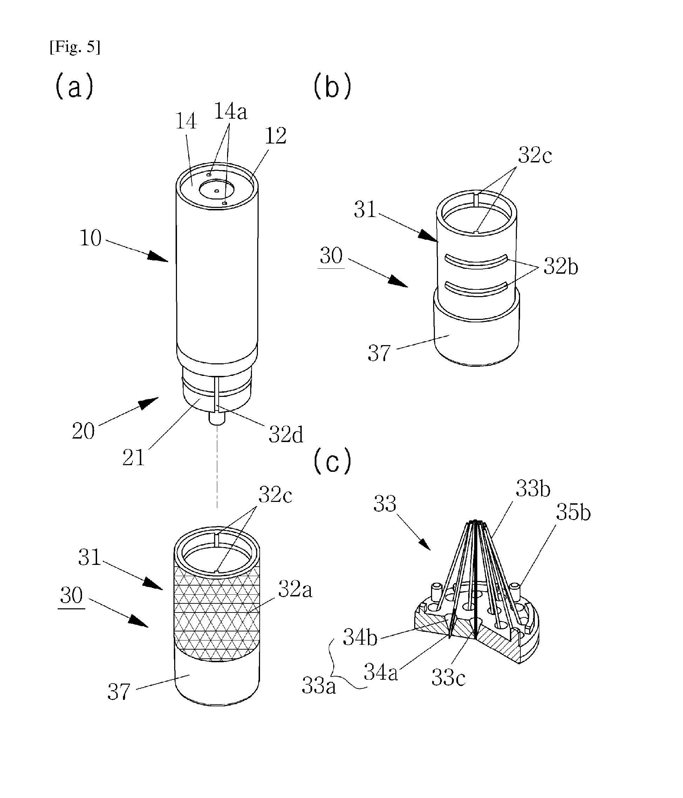

Referring to Figure 5A, the rubber grip 32a is formed by further attaching a flexible rubber material to the outer circumferential surface and is configured to guide for the user to smoothly grab while preventing slip. The above rubber grip 32a may be embossed in a vertical, horizontal or lattice-shape and may be as a whole formed in a shape of curved surfaces.

Referring to Figure 5B, the protrusion 32b is formed by integrally forming a protruding portion which is engaged with the user's fingers and is configured to guide for the user to easily grab in safe with the hands when grabbing while preventing slip. In addition to the illustrated configuration, the above protrusion 32b may be formed in such a way that small V-grooves are continuously formed in a X-shape and may be as a whole formed in a shape of curved surfaces.

Meanwhile, the rubber grip 32a and the protrusion 32b may be independently formed, but may be formed together so as to secure more improved grabbing feeling while preventing slipping. In any case, a person having ordinary skill in the art may provide various patterns and shapes and may further attach the same within a range where the grabbing feeling can be improved while preventing the slipping.

In addition, the housing 31 of the present invention further includes one or more than one fixing protrusion 32c and slide groove 32d on the top of the inner circumferential surface so as to prevent any rotations by means of the pumping unit 20. Referring to Figure 5A, the housing 31 is secured to the pumping unit 20 and performs up and down movements and includes a fixing protrusion 32c and a slide groove 32d so as to prevent the rotations by means of the pumping unit 20.

The fixing protrusion 32c is formed at an inner circumferential surface of the housing 31, and the slide groove 32d is formed at an outer circumferential surface of the airless pump 21. Namely, when the housing 31 and the airless pump 21 are engaged with each other, the fixing protrusion 32c and the slide groove 32d become engaged with each other, so the up and down movements are possible, but the rotations are impossible. Therefore, since the rotations are prevented in a state where the needle 33b remains penetrated in the skin 1, it is possible to inhibit the needle 33b from hurting the skin 1.

Referring to Figures 3 and 4, the needle unit 33 is configured to inject the solution 2 discharged from the airless pump 21, into the skin 1 and is tightly combined with the bottom of the housing 31. Namely, referring to Figure 3, the needle unit 33 includes a plurality of needle grooves 33a formed in a radial shape inside of the same, wherein the needle groove 33a serves to tightly fix the needle 33b, and the needle 33b serves to inject the discharging solution into the skin 1. The above mentioned needle unit 33 is characterized in that the needle 33b passes from the top through the needle groove 33a and is fixed in a state where it protrudes from the bottom by about 0.25∼0.6mm long and penetrates into the epidermis 1a or the dermis 1b of the skin 1. Here, it is preferred that one end of the needle 33b protruding from the bottom of the needle unit 33 is formed to have a tip relatively sharp enough for the needle 33b to easily penetrate inside the skin 1. Such a needle 33b is formed in a hollow shape having a passage at its center like an injection needle for thereby directly injecting the solution 2 discharged from the airless pump 21, into the skin 1.

At this time, the needle groove 33a of the needle unit 33 according to the present invention includes a fixing groove 34a for fixedly inserting the needle 33b, and a guide groove 34b guiding for the needle 33b to be guided to the center in safe over the top of the fixing groove 34a. Referring to Figure 3, the needle groove 33a is divided into a fixing groove 34a and a guide groove 34b, wherein the fixing groove 34a is fixed in a vertical direction for the needle 33b to inject the solution 2 into the skin 1, and the guide groove 34b is slanted at a predetermined angle for the needle 33b to be bent in safe toward the center while corresponding to the slanted member 31b.

The slanted member 31b serves to guide for the needles 33b to distribute with the same quantity the solution 2 discharged from the airless pump 21. For this, it is most preferred that the needles 33b are collectively disposed just below the fixture 31a. Above all, since the needles 33b are positioned closest to the fixture 31a, the solution 2 from the airless pump 21 may be directly transferred to the needles 33b shortly after the solution 2 is discharged from the airless pump 21 for thereby minimizing any consumption of the solution 2.

Like this, when it needs to secure a state where the needle unit 33 is combined with the housing 31, the needles 33b, referring to Figure 4, should be bent to be collected toward the center, for which the guide groove 34b serves to guide for the needles 33b to be naturally bent in safe from the fixing groove 34a. If the needles are bent to correspond to the slanted member 31b in a state where only the needles 33b are fixed using the fixing groove 34a without additionally using the guide groove 34b, the passage of the needle 33b may be blocked due to the sudden bending. Therefore, the needle groove 33a is divided into the fixing groove 34a and the guide groove 34b so as to fix the needles 33b in the bent state.

In addition, the needle unit 33 of the present invention includes a plurality of needles 33b protruding by predetermined lengths long enough to penetrate into the skin 1, and any one of such needles 33b further includes a non-protruding needle 33c for the contact with the surface of the skin 1.

Referring to Figure 3, the needles 33b are fixed in a state where the needles 33b protrude by about 0.25∼0.6mm long from the top, through the needle grooves 33a and to the bottom surface and then penetrate into the epidermis 1a or the dermis 1b of the skin 1. Referring to Figure 5C, at least one needle 33c is configured to contact only the surface of the epidermis 1a of the skin 1. Since the needles 33b penetrate into the skin 1, the needles 33b may cause uneasy feeling to the user based on the user's character (various disorders) or the skin state (allergy or skin disease). On the other hand, since the needle 33c contacts with only the surface of the skin 1, it is possible to use the needles in safe even though there are any problems. Here, even though the needles 22b and the needles 33 are illustrated in combination, but any ones of the needles 22b and the needles 33 may be independently disposed.

As illustrated in Figures 3 and 4, the fixing unit 35 serves to tightly fix the needle unit 33 at the inner center of the housing 31 to communicate with the fixture 31a and the slanted member 31b. Namely, as illustrated in Figure 3, a shoulder accommodating the needle unit 33 is provided at one end of the slanted member 31b of the housing 31, and at least one fixing groove 35a and fixing protrusion 35b are provided so that the housing 31 and the needle unit 33 are tightly inserted at the engaging positions. Here, a fixing groove 35a is formed at the shoulder of the housing 31, and the fixing protrusion 35b is provided at the needle unit 33, but according to the situation, the fixing protrusion 35b may be formed at the shoulder of the housing 31, and the fixing groove 35a may be provided at the needle unit 33. It is preferred that the fixing groove 35a and the fixing protrusion 35b are formed with a predetermined error so that a tight insertion may be secured at the engaging portion.

At this time, the fixing unit 35 according to the present invention fills a hardener 36 so as to minimize the consumption of the solution by fixing the needles 33b at the slanted member 31b of the housing 31 and blocking the space. Referring to Figures 3 and 4, the fixing unit 35 serves to tightly fix the needle unit 33 at the inner center of the housing 31 through the fixing groove 35a and the fixing protrusion 35b and to communicate the needle unit 33 with the fixture 31a and the slanted member 31b. The hardener 36 is filled so as to completely fix the needles 33b in a state where the needle unit 33 is combined with the housing 31 and so as to minimize the consumption of the solution 2.

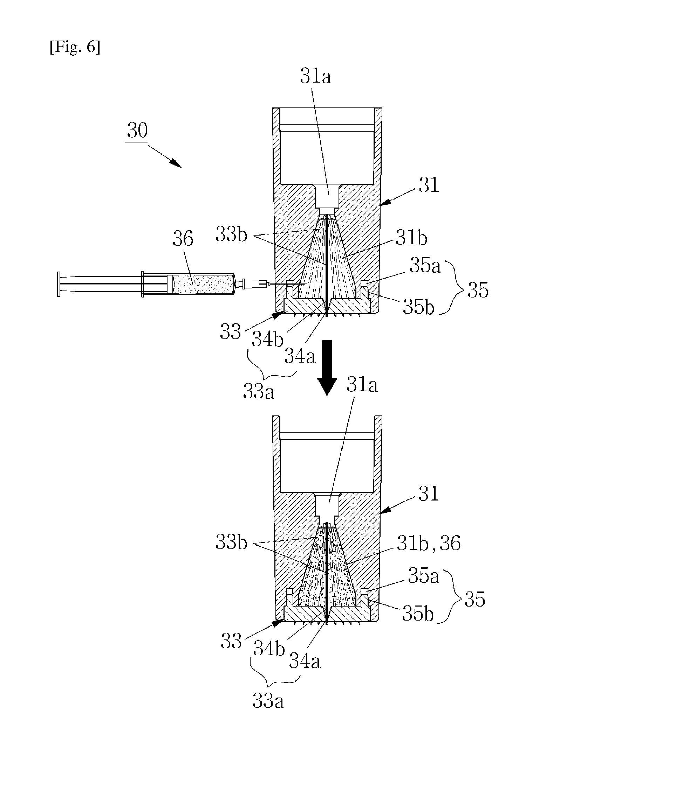

Namely, referring to Figure 6, an injection port (not illustrated) is formed, which communicates with the slanted member 31b from an outer circumferential surface of the housing 31 before or after the needle unit 33 is tightly engaged through the fixing groove 35a and the fixing protrusion 35b to the housing 31. The hardener 36 is filled through the injection port for thereby completely blocking the space of the slanted member 31b. Here, the hardener 36 is formed of a predetermined material which changes into a liquid state when heat with a predetermined temperature is supplied, and when the temperature gradually decreases, the hardener 36 change into a solid state. Here, the material of such a hardener 36 is not limited to a specific material, and various materials may be used.

When the hardener 36 is filled into the slanted member 31b of the housing 31, the needles 33b may be completely fixed, and the sealing state from the outside may be maintained. Above all, since the space of the slanted member 31b is completely blocked, the solution 2 discharged from the airless pump 21 may be directly transferred only to the needles 33b for thereby preventing any consumption of the solution 2.

Meanwhile, the housing 31 according to the present invention further includes a safety cap 37 with an engaging protrusion 37a being interposed so as to block any inputs of bacteria or impurities while protecting the needle unit 33. Referring to Figures 1 to 3, the safety cap 37 is combined with an outer circumferential surface of the housing 31 for thereby hiding, from the outside, the needles 33b protruding from the needle unit 33. Namely, since the needles 33b penetrate into the epidermis 1a or the dermis 1b of the skin 1, protecting (blocking) from bacteria or impurities is a very important factor. Therefore, it needs to protect the skin 1 from various infections in such a way to engage the safety cap 37 onto an outer circumferential surface of the housing 31 before and after the use.

The safety cap 37 includes engaging protrusions 31c and 37a at the inner and outer circumferential surfaces where the safety cap 37 is engaged with the housing 31 so as to increase the force of the engagement with the housing 31. Namely, when the safety cap 37 is inserted into the outer circumferential surface so as to protect the needles 33b exposed from the housing 31, the separation of the engaging protrusion 37a of the safety cap 37 is prevented by the engaging protrusion 31c of the housing 31. Of course, in addition to the above configuration, the engaging protrusion and the engaging groove may be formed at the inner and outer circumferential surfaces where the safety cap 37 and the housing 31 are engaged with each other, and any configuration wherein a tight fitting is secured using a predetermined error between the inner and outer circumferential surfaces without forming the engaging protrusions 31c and 37a of the housing 31 and the safety cap 37 is not excluded.

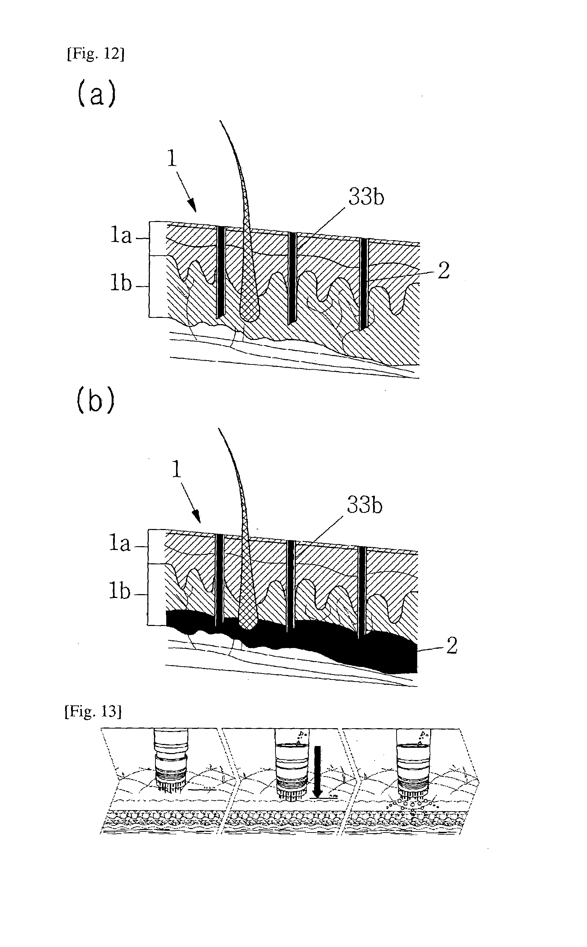

When in use based on the uses as in Figures 10 and 12, the injection mechanism is a tool which is capable of injecting the solution having predetermined efficacy into the epidermis 1a and the dermis 1b of the skin 1, but the injection mechanism may be provided in a state where it is combined with the pumping unit 20 wherein the solution 2 is filled in the accommodation unit 10 when manufacturing the product. In addition, the injection unit 30 may be provided in a state where it is separated from the pumping unit 20, and the safety cap 37 is coupled. Namely, the multiple pumping units 20 engaged with the accommodation unit 10 may be stored in the case (packing box), and the injection unit 30 may be vacuum-packed using a separate plastic material, etc. and may be provided in an encased state in the case together with the accommodation unit 10 and the pumping unit 20. Of course, all the accommodation unit 10 and the pumping unit 20 and the injection unit 30 may be manufactured in the assembled state along with the solution 2 and may be stored in the case and may be provided.

First, the user purchases the mechanism of the present invention filled with the solution 2 which has an effect good for skin improvements at a cosmetic sale shop or dermatology or a beauty-related shop. Thereafter, the user removes the packing of the injection unit 30 separately encased along with the accommodation unit 10 and the pumping unit 20 and assembles the pumping unit 20 and the injection unit 30. At this time, if the accommodation unit 10, the pumping unit 20 and the injection unit 30 all have be assembled and provided, the above-described procedures may be omitted.

As illustrated in Figure 10, the user presses downward the pumping unit to activate in a state where the injection unit 30 contacts with a predetermined portion of the skin 1 with one hand holding the accommodation unit 10. At this time, it is preferred that the user moves upwardly and downwardly the pumping unit 20 one to three times in a state where it is not in contact with the skin 1, so the solution 2 is fully filled into the needles 33b. Through the above-described procedures, the needles 33b are penetrated in the epidermis 1a and the dermis 1b of the skin 1 in the first stage as in Figure 12A.

The skin 1 of the human body is generally divided into the epidermis 1a and the dermis 1b. Although being different a little according to their positions, the epidermis 1a as a whole is about 0.25mm thick, and the dermis 1b is about 0.35 thick. Namely, the user does not feel any pain even though the needle 33b penetrates into the dermis 1b of the skin 1, but feels only a touch, so there is not any problem when in use. Here, since the skin 1 is generally influenced based on the substances residing at the dermis 1b, it is preferred that the needles 33b penetrate through the epidermis 1a to the portion where the dermis 1b is present.

Thereafter, as illustrated in Figure 10, when the pumping unit 20 starts to operate, the solution 2 of the accommodation unit 10 is discharged from the needles 33b and is penetrated into the dermis 1b in the second stage as in Figure 12B. Namely, as illustrated in Figure 10, the solution 2 is injected into a predetermined portion of the skin 1 where the user wants to. Though being different based on the volume of the accommodation unit 10, the solution may run out after the uses of about 20 to 50 times. Since the solution 2 is injected in a state where the solution 2 having predetermined efficacy is directly penetrated into the skin 1, about 90∼99% of absorption rate may be secured, which would result in reliable and good effects. Above all, it is possible to secure good effects with a small quantity of solution tanks to the high absorption rate, which minimizes the cost-bearing of the customer.

Meanwhile, the mechanism of the present invention the use of which is ended may be separated and discharged (discarded) for the purpose of recycling, but the accommodation unit 10, the pumping unit 20 and the injection unit 30 may be all separated and immersed in alcohol and may be sterilized by ultraviolet ray and may be dried, and then the accommodation unit 10 may be filled with the solution 2 and may be reused.

### Second Embodiment

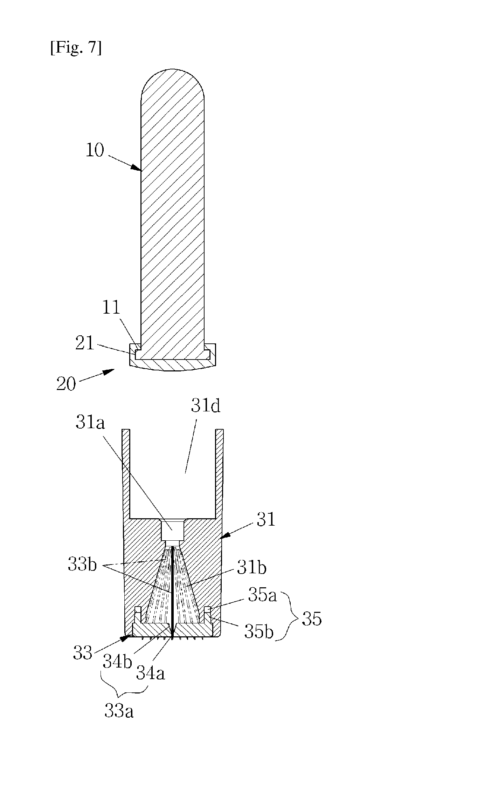

The handle unit 10 of the present invention is constituted in a protrusion shape for the user to grab and includes an engaging protrusion 11 which has at least on shoulders at the bottom. Referring to Figure 7, the handle unit 10 serves to guide for the solution 2 having outstanding effects for skin improvements and skin caring, to be discharged and injected into the skin 1. The handle unit 10 includes at its bottom an engaging protrusion 11 which protrudes by a predetermined length long enough for the user to easily grab.

As illustrated in Figure 7, the engaging protrusion 11 is guided to engage with the packing unit 20 which will be described later. Since the engaging protrusion 11 may be attached in engagement with the packing unit 20, but may be detached, if necessary, it is preferred that the engaging protrusion 11 includes at least one shoulder. Namely, the engaging protrusion 11 may be formed in various structures within a range where the packing unit 20 would not be disengaged from the engaged state.

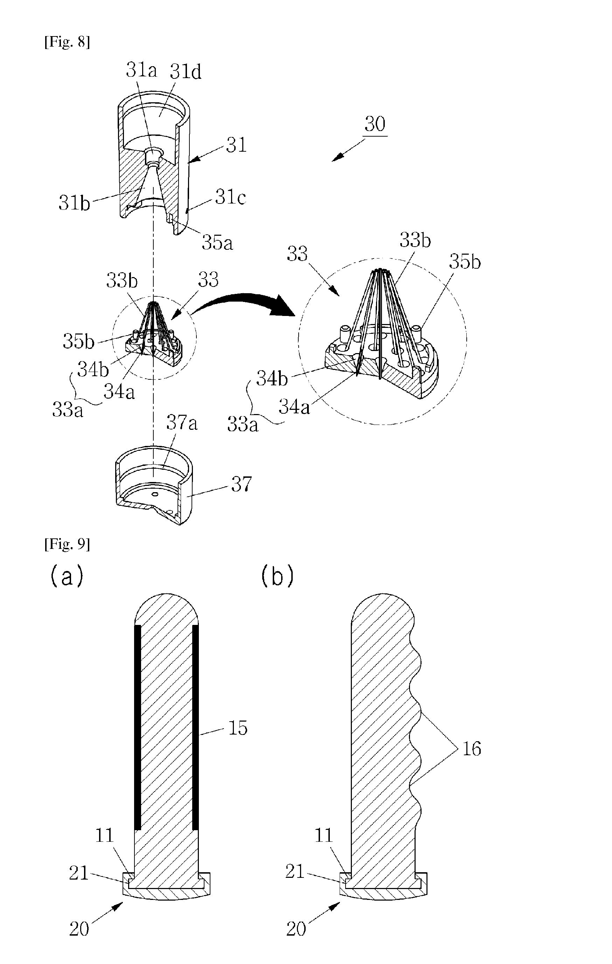

At this time, the handle unit 10 according to the present invention includes at its outer circumferential surface a rubber grip 15 or a protrusion 16 which makes it possible to prevent slip while improving grip feeling by a user. The handle unit 10 serves to inject the solution 2 into the skin 1 in cooperation with the packing unit 20 and the injection unit 30. As illustrated in Figure 9, a rubber grip 15 or a protrusion 16 is formed on an outer circumferential surface so that a pressurizing motion may be easily performed against the skin 1 in a state where the user is grabbing with hands.

As illustrated in Figure 9A, the rubber grip 15 is made by further attaching a flexible rubber material to an outer circumferential surface and serves to guide for the user to softly grab with a predetermined cushion and to prevent slip. In addition to the configuration as illustrated, the rubber grip 15 may be embossed in a vertical shape, a horizontal shape or a lattice shape or mat be formed as a whole in a shape of curved surfaces.

Referring to Figure 9B, the protrusion 16 includes, on its outer circumferential surface, integrally protruding portions engaged with the user's fingers and serves to guide for the user to grab in safe when grabbing with hands without any problems and to prevent any splitting. In addition to the configuration illustrated, the above protrusion 16 may be formed by continuously formed small V-grooves in a X-shape and may be as a whole formed in a shape of curved surfaces.

Meanwhile, the rubber grip 15 and the protrusion 16 may be independently formed, but may be formed in combination so as to improve grip feeling and prevent slip. In any case, a person having ordinary skill in the art may form various shapes and patterns within a range wide enough to improve grip feeling and prevent slip or more rubber grip or protrusion may be attached.

The packing unit 20 according to the present invention is detachably disposed at the bottom of the handle unit 10 and includes an engaging groove 21 which is fixedly engaged with the engaging protrusion 11. As illustrated in Figure 7, the packing unit 20 is configured to generate a predetermined pressure to inject the solution into the skin 1 in cooperation with the handle unit 10, and one side of the same is engaged with the engaging protrusion 11 for thereby securing attachment and detachment. Namely, the packing unit 20 is as a whole made from a rubber material and seals for the solution 2 to be stored into the injection unit 30 and generates pressure to the solution 2 by receiving a pressurizing motion of the handle unit 10 and discharges (injection) to the outside. The packing unit 20 is basically made from a material having good sealing force, and it is preferred that the packing unit 20 is made from a material which is harmless and not affected by the compositions of the solution 2.

In addition, the injection unit 30 of the present invention is sealingly engaged with the packing unit 20, and discharges the solution 2 and penetrates into the skin 1 based on the pressurizing motion of the handle unit 10. As illustrated in Figures 7 and 8, the injection unit 30 discharges the solution 2 and penetrates the same into the skin 1 based on the pressurizing motion of the handle unit 10 and is constituted to cooperate with the packing unit 20 while accommodating the solution 2.

At this time, the injection unit 30 according to the present invention includes a housing 31 which is combined with the packing unit 20 and includes a fixture 31a, a slanted member 31b and a space 31d for accommodating the solution 2, a needle unit 33 which includes needle grooves 33a and needles 33b at the bottom of the housing 31, and a fixture 35 which includes a fixing groove 35a and a fixing protrusion 35b for fixing the housing 31 and the needle unit 33.

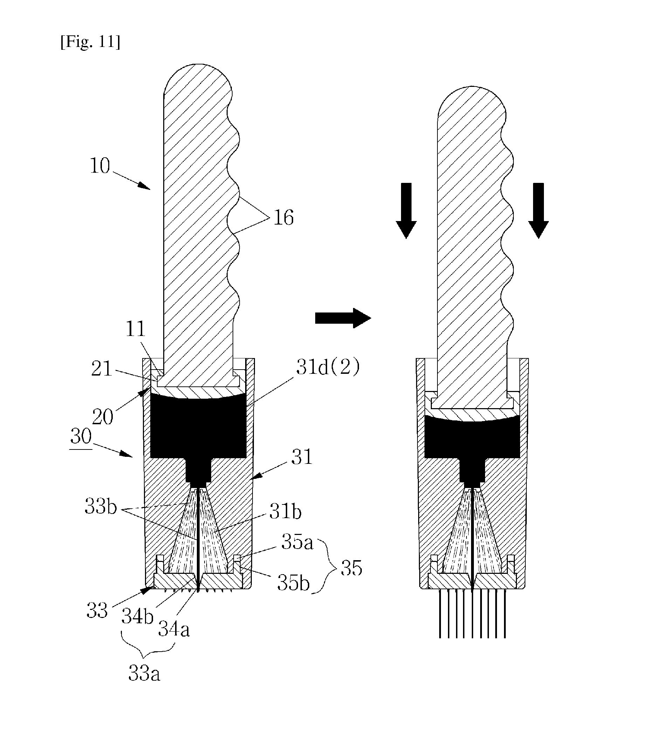

As illustrate in Figure8, the injection unit 30 has the same reference number as the injection unit 30 according to the first exemplary embodiment of the present invention, so the detailed operation and descriptions thereof will be omitted, provided that the space (not given the reference number) of the housing 31 according to the first exemplary embodiment is for the up and down motions in cooperation with the pumping unit 20, and the space 31d of the housing 31 according to the second exemplary embodiment is to accommodate the solution 2. Namely, in a state where the solution 2 is accommodated in the space 31d of the housing 31, as illustrated in Figure 11, the handle unit 10 combined with the packing unit 20 is inserted, and when the pressurizing motion is performed with respect to the skin 1 in a state where the user is holding the handle unit 10, as illustrated in Figure 12, the needles 23b penetrate into the skin 1, and at the same time the solution 2 is discharged with a predetermined pressure and is injected.

As described above, the present invention is constituted in a stamp type light, thin, short and small structure, so the manufacturing cost may be lowered, and every home may easily and conveniently use at a lower price. Since the solution may be uniformly and directly injected in a state where the needles are penetrated in the epidermis and dermis of the skin for thereby minimizing any loss of the solution and maximizing the effects.

The present invention is not limited to the disclosed exemplary embodiments, and it is obvious that a person having ordinary skill in the art might variously modify and change without departing from the concept and scope of the present invention. Therefore, such changes or modifications belong to the scope of the claims of the present invention.

### Legends of Reference Numbers

- 1:: skin
- 1a:: epidermis
- 1b:: dermis
- 2:: solution
- 10:: accommodation unit
- 11:: space
- 12:: suction port
- 13:: packing
- 14:: cover
- 14a:: suction hole
- 15:: discharge port
- 20:: pumping unit
- 21:: air less pump
- 30:: injection unit
- 31:: housing
- 31a:: fixture
- 31b:: slanted member
- 31c:: engaging protrusion
- 33:: needle unit
- 33a:: needle groove
- 33b:: needle
- 34a:: fixing groove
- 34b:: guide groove
- 35:: fixing unit
- 35a:: fixing groove
- 35b:: fixing protrusion
- 36:: hardener
- 37:: safety cap
- 37a:: engaging protrusion

## Claims

1. A self-injection mechanism for use on skin which is configured to inject a solution (2) having predetermined efficacy into a skin (1) for skin improvements or beauty treatment, comprising:
an accommodation unit (10) which includes a space (11) configured to accommodate the solution (2) inside the accommodation unit (10), a suction port (12) disposed at one end for sucking only air, and a discharge port (15) being open and disposed at the other end for discharging the solution (2);
a pumping unit (20) which is engaged to communicate with the discharge port (15) of the accommodation unit (10) and includes an airless pump (21) disposed at the center for discharging the solution (2) based on up and down motions; and
an injection unit (30) which is engaged to communicate with the airless pump (21) of the pumping unit (20) and is configured to penetrate the solution (2) discharged based on the up and down motions into the skin (1).

2. The mechanism of claim 1, wherein the accommodation unit (10) includes a cover (14) with a suction hole (14a) being interposed together with a packing (13) which floats over the space (11) while sealing the solution (2) of the suction port (12).

3. The mechanism of claim 1, wherein the injection unit (30) comprises:
a housing (31) which includes a fixture (31a) and a slanted member (31b) combined with the airless pump (21);
a needle unit (33) which includes a needle groove (33a) and a needle (33b) disposed at the bottom of the housing (31); and
a fixing unit (35) which includes a fixing groove (35a) and a fixing protrusion (35b) for fixing the housing (31) and the needle unit (33).

4. The mechanism of claim 1, wherein the housing (31) further comprises a rubber grip (32a) or a protrusion (32b) formed on an outer circumferential surface for improving grip feeling and preventing slip by a user.

5. The mechanism of claim 1, wherein the housing (31) further comprises at least one fixing protrusion (32c) and slide groove (32d) on the top of an inner circumferential surface for preventing the rotations from the pumping unit (20).

6. A self-injection mechanism for use on skin which is configured to inject a solution (2) having predetermined efficacy into a skin (1) for skin improvements or beauty treatment, comprising:
a handle unit (10) which protrudes for a user to grab and includes an engaging protrusion (11) having at least one shoulder at the bottom;
a packing unit (20) which is detachably disposed at the bottom of the handle unit (10) and includes an engaging groove (21) fixedly engaged with the engaging protrusion (11); and
an injection unit (30) which is sealingly engaged with the packing unit (20) and is configured to discharge and penetrate the solution (2) into the skin (1) based on the pressurizing motions of the handle unit (10).

7. The mechanism of claim 6, wherein the handle unit (10) comprises a rubber grip (15) or a protrusion (16) on its outer circumferential surface so as to improve grip feeling and prevent slip.

8. The mechanism of claim 6, wherein the injection unit (30) comprises:
a housing (31) which is combined with the packing unit (20) and includes a fixture (31a), a slanted member (31b) and a space (31d) for accommodating the solution (2);
a needle unit (33) which includes a needle groove (33a) and a needle (33b) at the bottom of the housing (31); and
a fixing unit (35) which includes a fixing groove (35a) and a fixing protrusion (35b) for fixing the housing (31) and the needle unit (33).

9. The mechanism of either claim 3 or claim 6, wherein the needle groove (33a) of the needle unit (33) comprises:
a fixing groove (34a) for fixedly inserting the needles (33b); and
a guide groove (34b) for guiding, in safe, for the needles (33b) to move to the center over the top of the fixing groove (34a).

10. The mechanism of either claim 3 or 6, wherein the needle unit (33) comprises a plurality of needles (33b) which protrude by predetermined distances long enough to penetrate into the skin (1), and at least one of the plurality of the needles (33b) is a non-protruding needle (33c) which comes into contact with the surface of the skin (1).

11. The mechanism of either claim 3 or 6, wherein the fixing unit (35) is configured to fix the needle (33b) onto the slanted member (31b) of the housing (31) and further fills a hardener (36) to minimize the consumption of the solution by blocking the space.

12. The mechanism of either claim 3 or 6, wherein the housing (31) further comprises a safety cap with an engaging protrusion (37a) being interposed so as to block the inputs of bacteria or impurities while protecting the needle unit (33).
